# EUROPÄISCHE PATENTSCHRIFT

(11) **EP 3 294 387 B1**
(45) Veröffentlichungstag und Bekanntmachung des Hinweises auf die Patenterteilung: **05.02.2020**
(21) Anmeldenummer: 16722653.9
(22) Anmeldetag: 12.05.2016
(51) Int. Cl.: A61M 5/46, A61K 38/48, A61K 9/00, A61M 5/34

(54) **AUFSATZ FÜR EINE ODER AN EINER VORRICHTUNG ZUM INJIZIEREN EINER FLÜSSIGKEIT IN ODER UNTER DIE HAUT**
ATTACHMENT FOR OR ON A DEVICE FOR INJECTING A FLUID INTO OR UNDER THE SKIN
ÉLÉMENT RAPPORTÉ POUR UN DISPOSITIF OU DESTINÉ À ÊTRE PLACÉ SUR LEDIT DISPOSITIF POUR L'INJECTION D'UN LIQUIDE DANS OU SOUS LA PEAU

(30) Priorität: 13.05.2015 DE 102015107613
(43) Veröffentlichungstag der Anmeldung: 21.03.2018
(73) Patentinhaber: Hahn-Schickard-Gesellschaft für angewandte Forschung e.V., 78052 Villingen-Schwenningen (DE)
(72) Erfinder: CLEMENZ, Markus, 78050 Villingen-Schwenningen (DE); KEGEL, Michael, 78144 Tennenbronn (DE)
(74) Vertreter: SR Huebner - Munich Patentanwaltspartnerschaft mbB
(86) Internationale Anmeldenummer: PCT/EP2016/060692
(87) Internationale Veröffentlichungsnummer: WO 2016/180924

(56) Entgegenhaltungen:
- EP-A1- 2 388 033
- EP-A2- 0 904 790
- WO-A1-2015/047114
- WO-A2-2009/107945

## Beschreibung

### Gebiet der Erfindung

Die Erfindung betrifft einen Aufsatz für eine oder an einer Vorrichtung zum Injizieren einer Flüssigkeit in oder unter die Haut, zum Beispiel von Botulinumtoxin in einen menschlichen Patienten.

### Hintergrund der Erfindung

Die Verabreichung von Botox (Botulinumtoxin) kann schwierig sein, da der behandelnde Arzt die Einstichtiefe der Spritze bei einem Einstichwinkel von zwischen 45° und 90° mit großem Geschick von Hand einstellen muss. Auf dem Gebiet dermaler Zugangsvorrichtungen, bei denen ein Einstich subkutan mit einem Einstichwinkel von weniger als 25° erfolgt, ist es aus der DE 10 2010 001 506 A1 bekannt, eine Kanüle für die Injektion der zu verabreichenden Flüssigkeit in zwei Stellungen zu verrasten, wobei bei der ersten Stellung die Kanüle weiter ausgefahren ist und die zweite Stellung durch Zurückfahren der Kanüle erreicht wird. Aus der WO 2009/107945 A2, der US 8,858,498 B2, der WO 2013/121307 A1, der DE 72 42 473 U und WO 2012/029082 A1 ist es bekannt, mehrteilige Aufsätze für Spritzen bereit zu stellen, wobei ein distales Teil gegenüber einem proximalen Teil beweglich ist, wobei durch die Bewegung ein Ausmaß des Herausragens einer Kanüle aus dem distalen Teil veränderbar ist.

Im Falle der Vorrichtung gemäß der WO 2009/107945 A2 ist ein distales Teil mit einem Innengewinde versehen und ein proximales Teil des Aufsatzes mit einem Außengewinde an seinem distalen Ende versehen. Das distale Teil wird auf das Außengewinde des proximalen Teiles aufgeschraubt. Vor dem Außengewinde an dem proximalen Teil ist eine Skala vorgesehen, die je nach Ausmaß ihrer Bedeckung unterschiedliche maximale Zahlenwerte sichtbar lässt. Nachteilig an dieser Anordnung ist der geringe Halt des distalen Teils, wenn die Kanüle nur wenig herausstehen soll und eine maximale Zahl der Skala ablesbar ist. Weiterhin nachteilig ist, dass keine diskreten Positionen einstellbar sind, und die Einstellung nur optisch zu kontrollieren ist und nicht taktil und akustisch. Weiterhin nachteilig ist, dass die Zahlenwerte auf der Skala nicht die Injektionstiefe angeben, sondern die Verkürzung der eingeschobenen Kanüle. Weiterhin nachteilig sind potenzielle Fehlermöglichkeiten, die sich durch die Möglichkeit ergeben, verschiedenen Kanülen für die Benutzung mit dem Gerät auszuwählen.

Bei dem Aufsatz der US 8,858,498 B2 sind zwei oder mehr stabile Positionen vorgesehen, die das distale Teil gegenüber dem proximalen Teil stabil einnehmen kann. Die WO 2013/121307 A1 zeigt mehrere stabile Raststellungen des distalen Teils. Im Falle der Stichtiefeneinstellung aus der DE 72 42 473 U ist es bekannt, auf einer Achse aufeinanderfolgend Sägezahnstrukturen vorzusehen, durch die der Aufsatz gegenüber einer Spritze stabil verrastbar ist. Die WO 2012/029082 A1 zeigt ebenfalls einen zweiteiligen Aufsatz für eine Spritze, wobei eine Nadel der Spritze beide Aufsatzteile durchdringt, wenn sich die Spritze an dem proximalen, inneren Aufsatzteil abstützt. Durch Drehung des proximalen Aufsatzteiles gegenüber dem distalen Aufsatzteil kann die Stichtiefe eingestellt werden. Auch hieran ist nachteilig eine geringe Stabilität der Anordnung.

Es ist ferner das Konzept bekannt, die Spritze im Ganzen gegenüber einem Aufsatz zu verschieben. Im Falle einer Einstichtiefeneinstellung aus der EP 2 523 707 B1 fährt beispielsweise eine Spritze für Insulin auf einem Schlitten gegenüber einem Gehäuse. Das Verfahren erfolgt über ein Getriebe, das einen großen Zahnkranz außerhalb des Schlittens und eine kleinen Zahnkranz auf einem drehbaren Zylinder als Teil des Schlittens umfasst. In der US 2001/0047151 A1 ist an einem Spritzenaufsatz eine Schraubenmutter vorgesehen, die zugleich als Sitz für eine Schürze an dem nadelseitigen Ende der Spritze vorgesehen ist. Die DE 200 135 79 U1 offenbart ferner ein nachfüllbares Dosier- und Injektionsgerät, bei dem mittels einer Stellmutter eine Einstichtiefe einer Kanüle festlegbar ist. Zur Regelung der Einstichtiefe einer Nadel ist es aus der WO 98/50094 A1 bekannt, die Nadel an einem Innenkörper zu halten, der mit einem Außengewinde versehen ist, das in einem Innengewinde eines äußeren Körpers verdrehbar ist. Die Verdrehung erfolgt durch Betätigung eines Stellrings. Die Nadel wird aus dem einen Ende herausgefahren und zugleich aus dem Flüssigkeitsreservoir für die zu injizierende Flüssigkeit herausgefahren. Das Flüssigkeitsreservoir kann gegebenenfalls durch manuelles Betätigen nachgeschoben werden. Nachteilig hieran ist, dass die gesamte Vorrichtung sehr komplex und fehleranfällig ist.

EP 0 904 790 A2 offenbart eine Stiftnadel, die einen Extender aufweist, der drehbar an einem Hub auf der Stiftnadel angebracht ist, um die Stiftnadel auf eine gewünschte Injektionslänge einzustellen. Der Extender kann in verschiedene vordefinierte Stellungen gebracht werden, wobei jede Stellung einer vordefinierten Injektionslänge entspricht.

EP 2 388 033 A1 offenbart einen an eine Spritze ankoppelbaren Rohraufsatz, der einen Entkopplungskörper und ein Steuerrohr umfasst. Das Steuerrohr wird an das obere Ende der Spritze angekoppelt und ist auf dem Kopplungskörper gleitend bewegbar. Das Steuerrohr weist auch ein Loch auf, durch das die Kanüle der Spitze treten kann.

### Der Erfindung zugrundeliegende Aufgabe

Der Erfindung liegt die Aufgabe zugrunde, eine zuverlässige technische Hilfe beim Zuführen von Botox in und unter die Haut eines menschlichen Patienten bereitzustellen.

### Erfindungsgemäße Lösung

Die der Erfindung zugrundeliegende Aufgabe wird durch einen Aufsatz für eine oder an einer Vorrichtung zum Injizieren einer Flüssigkeit in oder unter die Haut, zum Beispiel von Botulinumtoxin in einen menschlichen Patienten, nach Anspruch 1 gelöst, wobei der Aufsatz ein proximales Teil aufweist, das an die Vorrichtung ankoppelbar oder mit dieser verbunden ist, und das eine Kanüle trägt, wobei der Aufsatz ferner ein distales Teil aufweist, das die Kanüle zumindest abschnittsweise umgibt und wobei das proximale Teil und das distale Teil derart zueinander drehbeweglich sind, dass ein Ausmaß des Herausragens der Kanüle aus dem distalen Teil veränderbar ist. Die Drehbeweglichkeit beinhaltet, dass bei der Drehbewegung von proximalem zu distalem Teil oder umgekehrt eine diskrete Anzahl von Haltestellungen erreicht werden kann. Eine Haltestellung ist eine Stellung, in der die Bewegung aus der Haltestellung heraus in zumindest eine Richtung durch einen Widerstand beschränkt ist. Der Widerstand ist Vorzugsweise durch Aufwenden von Kraft überwindbar.

Die Bewegung ist sowohl in eine Richtung beschränkt, die ein Hereinfahren der Kanüle in das distale Teil bewirkt, als auch in eine Richtung, die ein Herausfahren der Kanüle in das distale Teil bewirkt. Der Widerstand ist durch einen Rastmechanismus verwirklicht. Es gibt mindestens zwei, besonders mindestens drei, weiter vorzugsweise mindestens fünf, und im höchsten Maß vorzugsweise mindestens zehn solcher Haltestellungen, wobei beispielsweise pro Millimeter Ausfahrlänge der Kanüle aus dem distalen Teil zwischen einer und zwanzig, beispielsweise mindestens drei, vier, fünf oder zehn Haltestellungen durchlaufen werden. Es ist ein Rastmechanismus vorgesehen, der eine Mehrzahl von Raststellungen bereitstellt, denen ein unterschiedliches Ausmaß des Herausragens der Kanüle aus dem distalen Teil entspricht. Eine Raststellung im Sinne der vorliegenden Anmeldung kann durch Formschluss bereitgestellt sein, alternativ aber auch mittels Kraftschluss erzielt werden, z.B. ist durch eine lokale Änderung des Materials oder der Oberflächenrauigkeit erzielbar, dass ein Reibwiderstand variiert. Die Rasteinrichtung weist eine oder mehrere Nut(en) und mindestens eine in die Nut(en) eingreifendes elastisches Rastglied auf.

Mit der Erfindung ist es dem behandelnden Arzt möglich, die Kanüle über eine definierte Länge aus dem distalen Teil herausragen zu lassen. Diese Länge entspricht der späteren Einstichtiefe, wenn eingestochen wird, bis das distale Teil, beispielsweise mit einer geeignet ausgebildeten Stirnfläche, die Haut berührt. Durch das Vorsehen der Haltestellungen ist dem behandelnden Arzt eine haptische oder taktile und gegebenenfalls auch akustische Erfassung der Ausfahrlänge der Kanüle ermöglicht. Ferner kann bei bereits in den menschlichen Patienten eingestochener Kanüle noch eine Nachregelung möglich sein, indem die nächste oder eine weitere Raststellung durch Drehen nunmehr des proximalen Teils gegenüber dem distalen Teil eingenommen wird. Ebenso kann auch ein Herausfahren durch Drehung in Gegenrichtung möglich sein.

### Bevorzugte Ausgestaltung der Erfindung

Vorteilhafte Aus- oder Weiterbildungen, welche einzeln oder in Kombination miteinander eingesetzt werden können, sind Gegenstand der abhängigen Ansprüche betreffend den ersten Aspekt der Erfindung.

Vorzugsweise ist bei dem Aufsatz vorgesehen, dass die Raststellungen beim Drehen des distalen Teiles in dieselbe Drehrichtung sukzessive durchlaufen werden und sich hierbei das Ausmaß des Herausragens der Kanüle entweder ständig weiter vergrößert oder ständig weiter verkleinert. Eine solche Lösung ist mechanisch sehr einfach umsetzbar.

Der Aufsatz umfasst einen Zahnkranz aus axial verlaufenden Zähnen mit dazwischen liegenden axial verlaufenden Nuten und weist ferner ein elastisches Rastglied auf, das in einer Raststellung in eine der Nuten eingreift. Somit kann der Zahnkranz gedreht werden, wenn das proximale oder wenn das distale Teil gedreht wird. Die Raststellungen werden dann beim Drehen auf natürliche Weise durchlaufen. Wird das elastische Rastglied - wie vorzugsweise vorgesehen - als metallene Lamelle ausgebildet, ist es einerseits sehr einfach bereitzustellen, sorgt aber andererseits für eine stabile Verrastung.

Wenn der Zahnkranz an dem proximalen Teil ausgebildet ist und er näher an der Kanüle angeordnet ist als ein Gewinde, das die Drehbewegung des distalen Teils gegenüber dem proximalen Teil ermöglicht, dann ist erreichbar, dass das distale Teil stabiler an dem proximalen Teil gehalten ist als wenn das Gewinde weiter vorne, das heißt, weiter distal, ausgebildet ist. Zudem ist die Verrastung stabiler, wenn sie im Nahbereich der Kanüle und damit des Patienten erfolgt.

Wenn der Zahnkranz an einer Innenseite des distalen Teiles ausgebildet ist, ist erreichbar, dass eine größere Anzahl von Rastpositionen bei vorgegebenem Außenumfang des Aufsatzes eingenommen werden können als wenn der Zahnkranz an der Außenseite des proximalen Teils ausgebildet ist.

Der bevorzugte erfindungsgemäße Aufsatz umfasst außen an dem proximalen Teil eine Spiralnut, in die das distale Teil eingreift. Auf diese Weise ist erreichbar, dass das distale Teil das proximale Teil als Ganzes umgeben und zur Montage einfach aufgesetzt werden kann. Weiter bevorzugt ist es, dass bei an dem proximalen Teil ausgebildetem Zahnkranz, der näher an der Kanüle ist als es die Spiralnut ist, das distale Teil und das proximale Teil jeweils von kreisrundem Querschnitt sind, und der Zahnkranz eine maximale radiale Erstreckung hat, welche kleiner ist als der radiale Abstand eines Grundes der Spiralnut. Mit anderen Worten ist das proximale Teil an seinem distalen Ende weniger dick als an der Vorrichtung zum Injizieren von Flüssigkeit zugewandten Ende. So wird durch eine feine, dünne Spitze ein Präzisionswerkzeug geschaffen, mit dem sehr fein punktiert werden kann, und das die Punktionsstelle während der Punktion nicht breit abdeckt, so dass die Injektionsflüssigkeit sehr gezielt und präzise verabreicht werden kann.

Die Spiralnut kann in vorteilhafter Weise derart ausgebildet sein, dass zwischen ihren verschiedenen Abschnitten ein eine Information tragender Abschnitt (also erhaben zwischen den Spiralnuten) angeordnet ist, insbesondere ein Abschnitt mit einer Zahl, einem Buchstaben, einem Symbol, einem Skalenstrich, einem Grauwert aus einer Grauskala oder einer Farbe aus einer Farbskala oder einer Kombination aus einem oder mehreren dieser vorgenannten Elemente. Somit wird die Spiralnut über eine größere axiale Länge gestreckt, wo mit weniger Drehungen ein größerer axialer Bereich durch das distale Teil durchlaufen werden kann, die Kanüle also mit relativ wenigen Drehungen freigelegt und verlängert werden kann bzw. umgekehrt verkürzt werden kann, was das Ausmaß ihres Herausragens angeht. Diese Ausführungsform kann durch geeignetes Vorsehen des Rastmechanismus mit einer Mehrzahl oder gar Vielzahl von Raststellungen optimal ausgebildet werden. Als Information ist vorzugsweise die jeweilige Einstichtiefe beziehungsweise das Ausmaß des Herausragens der Kanüle aus dem distalen Teil ablesbar.

Der bevorzugte erfindungsgemäße Aufsatz umfasst alternativ zu der Spiralnut an dem proximalen Teil außen in einem inneren Hohlraum des proximalen Teils ein Innengewinde, in welches ein Schaft des distalen Teils eingreift. In diesem Fall lässt sich die Außenfläche des proximalen Teils freier gestalten.

Als Alternative zu dem Zahnkranz aus axial verlaufenden Zähnen kann eine Mehrzahl von Zähnen mit Zwischenräumen (Nuten) bereitgestellt sein, wobei ein elastisches Rastglied in einen Zwischenraum (Nut) eingreift, wenn es eine Raststellung einnimmt, wobei es in axialer Richtung elastisch bewegbar ist. Die elastische Beaufschlagung in axialer Richtung sorgt in erhöhtem Maße für eine Stabilität beim Injizieren.

Bei einer dritten Alternative ist unter Verzicht auf einen gesonderten Zahnkranz im Bereich einer außen an dem proximalen Teil vorgesehene Spiralnut, in die das distale Teil eingreift, eine Mehrzahl von Rastvertiefungen vorgesehen, wobei diese konkav und rund sein können oder als eckige Aussparungen bzw. Vertiefungen ausgebildet sein können. Diese Ausführungsform hat den Vorteil einer hohen Kompaktheit.

Gemäß einem zweiten Aspekt der Erfindung ist ein Aufsatz für eine oder an einer Vorrichtung zum Injizieren einer Flüssigkeit in oder unter die Haut, zum Beispiel von Botulinumtoxin in einen menschlichen Patienten bereitgestellt, wobei der Aufsatz ein proximales Teil aufweist, welches an die Vorrichtung ankoppelbar oder mit dieser verbunden ist, und das eine Kanüle trägt, wobei das proximale Teil in einem Abschnitt eine Spiralnut auf seiner Außenseite aufweist. In die Spiralnut greift ein distales Teil ein, welches die Kanüle zumindest abschnittsweise umgibt, wobei das proximale Teil und das distale Teil derart zueinander drehbeweglich sind, dass das Ausmaß des Herausragens der Kanüle aus dem distalen Teil veränderbar ist. Es sind axial aufeinanderfolgend Abschnitte der Spiralnut durch solche erhabenen Abschnitte des proximalen Teils voneinander beabstandet, welche eine Information tragen, die mit Hilfe von Zahlen, Buchstaben, Symbolen, Skalenstrichen und/oder einer Grau- oder Farbskala angegeben ist oder einer Kombination aus einem oder mehreren dieser vorgenannten Elemente. Als Information ist vorzugsweise die jeweilige Einstichtiefe beziehungsweise das Ausmaß des Herausragens der Kanüle aus dem distalen Teil ablesbar. Mit anderen Worten ist hier vorgesehen, dass die Spiralnut bei nur geringer Drehung eine relativ große axiale Strecke durchläuft. Um den Platz optimal zu nutzen, ist eben die Skala, welche insbesondere die Information wiedergibt, wie weit die Kanüle heraussteht, zwischen den Abschnitten der Spiralnut vorgesehen.

Vorzugsweise ist insbesondere vorgesehen, dass sich die Spiralnut über einen axialen Abschnitt von zwischen 20 % und 60 % der axialen Gesamterstreckung eines die Kanüle tragenden Körpers des proximalen Teils erstreckt. Mit anderen Worten wird ein sehr großer Anteil des proximalen Teils dazu verwendet, die Spiralnut bereitzustellen, wodurch die Einstichtiefe (das Ausmaß des Herausragens der Kanüle) im Verhältnis zur Gesamtgröße des Aufsatzes sehr stark variiert werden kann.

Gemäß einem dritten Aspekt der Erfindung ist ein Aufsatz für eine oder an einer Vorrichtung zum Injizieren einer Flüssigkeit in oder unter die Haut, insbesondere von Botulinumtoxin in einen menschlichen Patienten, bereitgestellt, wobei der Aufsatz ein proximales Teil aufweist, das an die Vorrichtung ankoppelbar oder mit dieser verbunden ist, das eine Kanüle aufweist und in einem Abschnitt eine Spiralnut auf seiner Außenseite aufweist. In die Spiralnut greift ein distales Teil ein, das die Kanüle zumindest abschnittsweise umgibt. Das distale Teil und das proximale Teil sind derart zueinander drehbeweglich, dass das Ausmaß des Herausragens der Kanüle aus dem distalen Teil veränderbar ist. Die Spiralnut erstreckt sich über einen axialen Abschnitt von zwischen 20 % und 60 % der axialen Gesamterstreckung eines die Kanüle tragenden Körpers des proximalen Teils. Hier wird somit ein sehr großer Anteil des proximalen Teils für die Spiralnut genutzt, so dass im Verhältnis zur Gesamtgröße des Aufsatzes das Ausmaß des Herausragens der Kanüle sehr stark variiert werden kann.

Bei dem Aufsatz gemäß allen drei Aspekten ist vorzugsweise eine Einrichtung an dem distalen Teil vorgesehen, mit Hilfe derer das distale Teil an dem proximalen Teil in einer Stellung festhaltbar ist, wobei es sich hierbei vorzugsweise um eine solche Stellung handelt, in der die Kanüle nicht aus dem distalen Teil herausragt. So wird für einen Nadelstichschutz vor der Injektion beim Aufsetzen der Vorrichtung zum Injizieren oder auch nach dem Einfahren der Kanüle und bei einem Wechsel der Vorrichtung zum Injizieren gesorgt. Durch diese Einrichtung - etwa in Form einer Arretierungstaste - ist vorzugsweise ein noch stabilerer Halt in der Stellung erzielbar als mit Hilfe des Rastmechanismus.

Gemäß einem vierten Aspekt ist ein Aufsatz für eine oder an einer Vorrichtung zum Injizieren einer Flüssigkeit in oder unter die Haut, zum Beispiel von Botulinumtoxin in einen menschlichen Patienten bereitgestellt, wobei der Aufsatz ein proximales Teil aufweist, das an die Vorrichtung ankoppelbar oder mit dieser verbunden ist, und das eine Kanüle trägt. Ein distales Teil umgibt die Kanüle zumindest abschnittsweise und ist gegenüber dem proximalen Teil translatorisch beweglich, so dass ein Ausmaß des Herausragens der Kanüle aus dem proximalen Teil veränderbar ist. Eine Stellschraube greift in ein Gewinde an dem proximalen oder dem distalen Teil ein und weist außerhalb des anderen Teils einen Griff, beispielsweise mit einer Riffelung, zum Angreifen auf. Beim Vorsehen einer solchen Stellschraube zum Verfahren des distalen Teils gegenüber dem proximalen Teil ist durch die Schraube selbst für einen stabilen Halt gesorgt.

Bei allen vier genannten Aspekten des erfindungsgemäßen Aufsatzes umfasst vorzugsweise das distale Teil zumindest einen Abschnitt aus einem elastischen nachgebenden Material, so dass durch einen Benutzer das distale Teil soweit zusammendrückbar ist, dass das proximale Teil fassbar ist. Dadurch ist die Handhabung erleichtert.

In einer Ausführungsform umfasst das distale Teil eine Stirnfläche auf, welche flach ist. Alternativ kann eine solche Stirnfläche vorhanden sein, welche gewölbt ist, zum Beispiel im Ganzen konvex oder im Ganzen konkav ist. Durch eine flache oder gewölbte Stirnfläche ist es möglich, das distale Teil stabil auf die Haut eines menschlichen Patienten aufzusetzen, wobei sie insbesondere einen geeigneten Endpunkt der Injizierbewegung darstellt. Der für eine Injektion von Botox bevorzugte Einstechwinkel von zwischen 45° und 90° kann bei geeigneter Ausbildung der Stirnfläche für den behandelnden Arzt leichter erzielbar sein. Weiter alternativ kann das distale Teil an seinem distalen (freien) Ende spitz ausgebildet sein, vorzugsweise konisch zulaufen, was eine Sichtkontrolle beim Einstechen erleichtert.

Bei einer bevorzugten Ausführungsform in allen vier genannten Aspekten ist das distale Teil zumindest an seinem distalen Ende abschnittsweise (z.B. nur über einen Teilumfang) oder vollständig transparent. Dadurch kann beim Injizieren der Kanüle in die Haut und nachfolgend in den Gesichtsmuskel eine Sichtkontrolle durch den behandelnden Arzt erfolgen.

Ferner ist bei allen Aspekten der Erfindung vorzugsweise in dem distalen Teil ein transparentes Fenster, gegebenenfalls mit einem Vergrößerungsglas, eine Öffnung oder auch eine elektrisch betriebene Anzeigevorrichtung vorgesehen. Durch das Fenster, das Vergrößerungsglas oder die Öffnung ist eine darunter liegende Skala, insbesondere auf dem proximalen Teil, ablesbar. Die elektrisch betriebene Anzeige kann ebenfalls eine Information über die Einstichtiefe (bzw. das Ausmaß des Herausragens der Kanüle aus dem distalen Teil) geben.

Der Aufsatz kann auf verschiedene Weise bereitgestellt sein.

Der Aufsatz kann auf seinem proximalen Teil eine Kupplung zum Ankoppeln an die Vorrichtung zum Injizieren an der Flüssigkeit aufweisen, z.B. in Form eines Luer Lock®, eines Luer Slip®, eines Konus zum Ankoppeln einer Spritze oder eines Schlauchanschlusses. Dadurch kann der Aufsatz gesondert bereitgestellt werden und flexibel an unterschiedliche Arten von Vorrichtungen zum Injizieren einer Flüssigkeit angekoppelt werden.

Alternativ kann der Aufsatz integral mit der Vorrichtung zum Integrieren einer Flüssigkeit sein, so dass der behandelnde Arzt ihn nicht selbst anbringen muss und die Vorrichtung zum Injizieren einer Flüssigkeit mit dem Aufsatz kompakt lagerbar ist.

Weiter alternativ kann der Aufsatz in einem Ultraschallkopf für ein Ultraschallgerät / eines Ultraschallgerätes integriert sein.

Schließlich kann auch der Aufsatz in oder an einem Dosier-Regler-Aufsatz für eine Vorrichtung zum Injizieren einer Flüssigkeit ausgebildet sein, d.h. man kann gleich zwei Aufsatzeinheiten gemeinsam vorsehen.

### Kurzbeschreibung der Figuren

Die Erfindung wird im Folgenden anhand schematischer Zeichnungen in weiteren Einzelheiten näher erläutert. Es zeigen:
- Fig. 1:: einen Aufsatz gemäß einem ersten Ausführungsbeispiel in perspektivischer, teilweise offener Ansicht, wobei die Kanüle nicht aus dem Aufsatz hervorsteht;
- Fig. 2:: den Aufsatz aus Fig. 1 in einer Stellung, in der die Kanüle um einen Abstand d₃ herausragt;
- Fig. 3:: eine Explosionsansicht des Aufsatzes aus Fig. 1;
- Fig. 4:: den Aufsatz aus Fig. 1, abgeschnitten an der Linie IV-IV in perspektivischer Ansicht;
- Fig. 5:: den Schnitt IV-IV aus Fig. 1;
- Fig. 6:: den Aufsatz aus Fig. 1 mit daran angesetzter Spritze, wobei der Aufsatz in einem Winkel von 90° auf einer eine Patientenhaut schematisch darstellenden Ebene steht;
- Fig. 7:: den Aufsatz aus Fig. 6, nur um einen Winkel von kleiner als 45° gedreht;
- Fig. 8:: einen perspektivischen Teilausschnitt des proximalen Teils mit einer zum distalen Teil gehörigen Arretierungstaste gemäß einer ersten Ausführungsform in einer Verriegelungsstellung und
- Fig. 9:: die Darstellung aus Fig. 8 in entriegelter Stellung;
- Fig. 10:: einen perspektivischen Teilausschnitt des proximalen Teils mit einer zum distalen Teil gehörigen Arretierungstaste gemäß einer zweiten Ausführungsform in einer Verriegelungsstellung und
- Fig. 11: die Darstellung aus Fig. 10 in entriegelter Stellung;
- Fig. 12:: einen Aufsatz gemäß eines zweiten Ausführungsbeispiels in perspektivischer, teilweise offener Ansicht, wobei die Kanüle nicht aus dem Aufsatz hervorsteht;
- Fig. 13:: eine Explosionsdarstellung des Aufsatzes aus Fig. 12;
- Fig. 14:: einen Aufsatz gemäß eines dritten Ausführungsbeispiels in perspektivischer, teilweise offener Ansicht, wobei die Kanüle nicht aus dem Aufsatz hervorsteht;

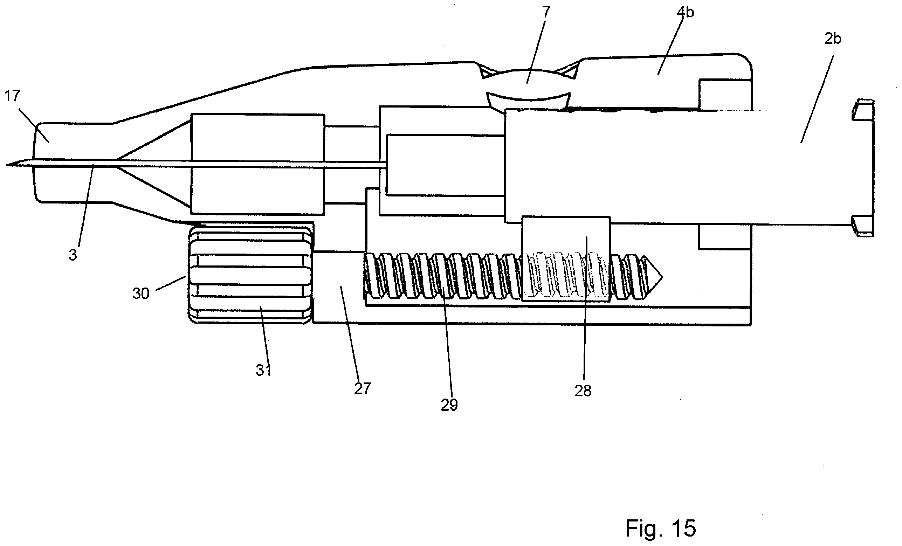

- Fig. 15:: den Aufsatz aus Fig. 14 im Längsschnitt XV-XV;
- Fig. 16:: einen Dosier-Regler-Aufsatz bei dem die Erfindung in einem Aspekt ebenfalls verwirklicht ist.

### Ausführliche Beschreibung der Erfindung anhand von Ausführungsbeispielen

Das Verabreichen von Botulinumtoxin (Botox) mithilfe einer Spritze (Verfahrkolben in Flüssigkeitsreservoir) soll erleichtert werden, und zwar soll insbesondere zunächst die präzise Tiefeneinstellung der Injektion erleichtert und ein zu flaches oder zu tiefes Einstechen verhindert werden. Ferner sollen mehrere, schnelle, repetitive Injektionen hintereinander in die gleiche Tiefe erleichtert werden.

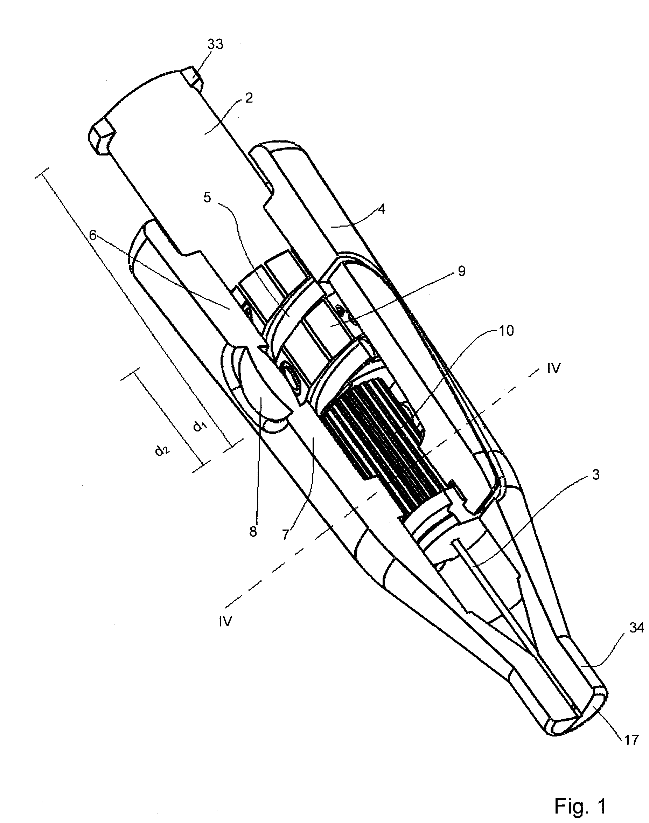

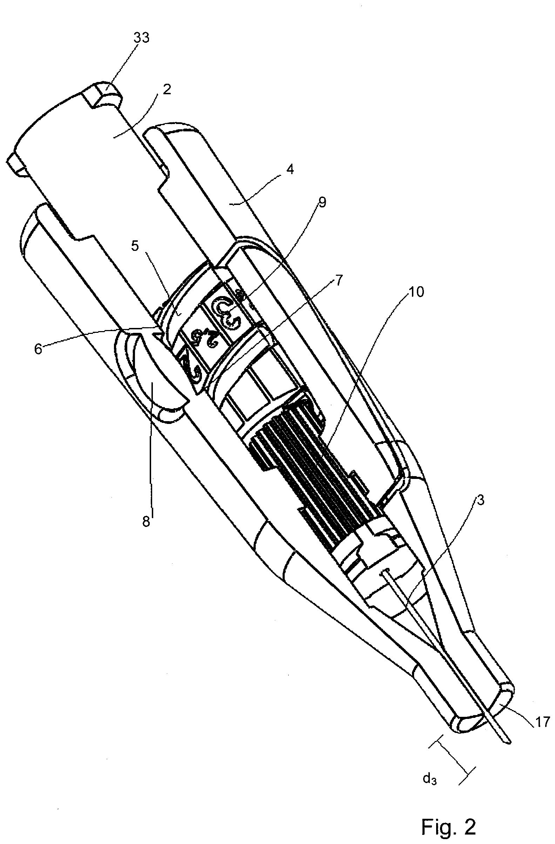

Fig. 1 zeigt ein erstes Ausführungsbeispiel eines erfindungsgemäßen Aufsatzes 1 mit einem proximalen Teil 2, welches eine Kanüle 3 trägt, und mit einem distalen Teil 4. Die Kanüle 3 ist durch das gesamte proximale Teil axial geführt und steht an dessen distaler Seite heraus. Das distale Teil 4 umgibt das proximale Teil 2 und insbesondere auch die Kanüle 3 zumindest abschnittsweise, also über zumindest einen Teilumfang und/oder eine in axialer Richtung gemessene Teillänge. Das proximale Teil 2 umfasst eine Spiralnut 5, in die zwei Führungsspitzen oder -zapfen 6 und 7 des distalen Teils 4 eingreifen. Zwischen den Führungsspitzen 6 und 7 befindet sich eine Vergrößerungslinse 8, durch die hindurch ein zwischen zwei Abschnitten der Spiralnut 5 befindlicher erhabener Teil 9, der eine Skala, vorliegend in Form von Zahlwerten, trägt, sichtbar ist. Die Spiralnut 5 erstreckt sich über eine Gesamtlänge d₂ eines Grundkörpers des proximalen Teils 2 mit der axialen Erstreckung d₁ und ermöglicht bei einem Gleiten der Führungsspitzen 6 und 7 in der Spiralnut 5 ein Herausfahren der Kanüle 3 um eine Länge d₃, siehe Fig. 2. Die Skala ist derart gestaltet, dass durch die Vergrößerungslinse 8 als Information eine Angabe der jeweiligen Einstichtiefe ablesbar ist.

Das Verhältnis von d₂ zu d₁ beträgt 40 %.

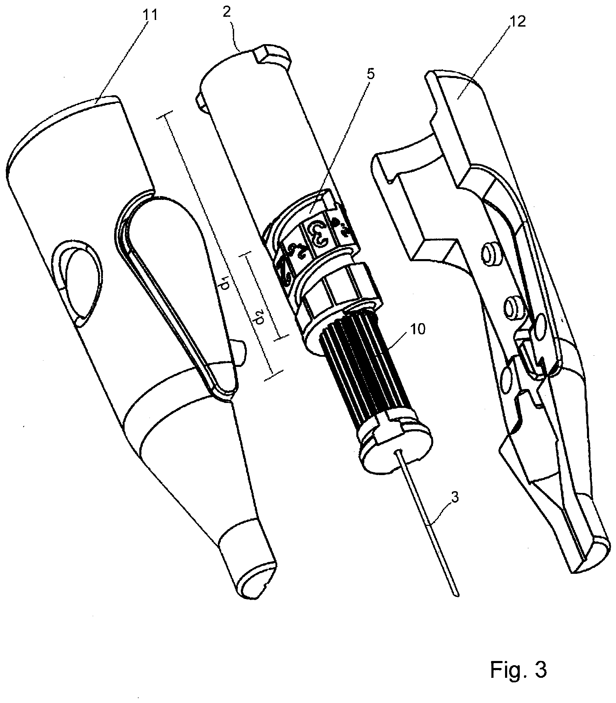

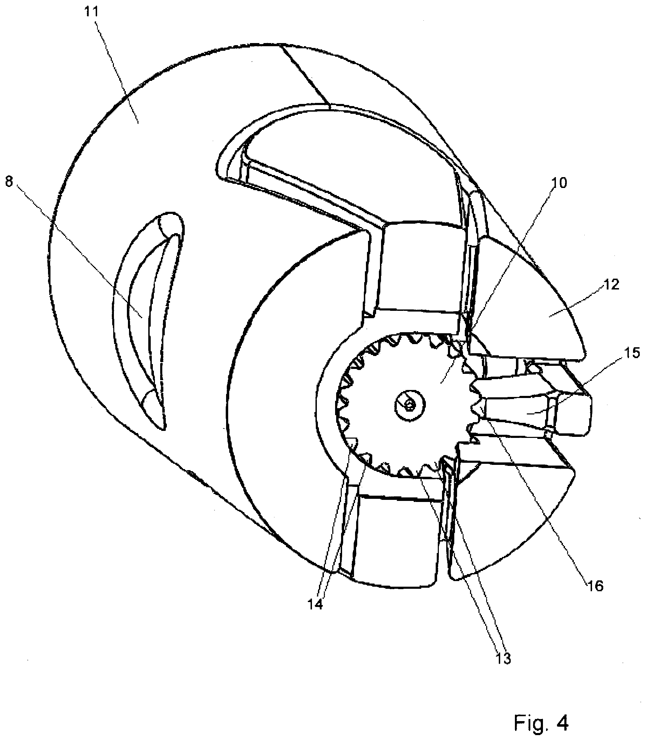

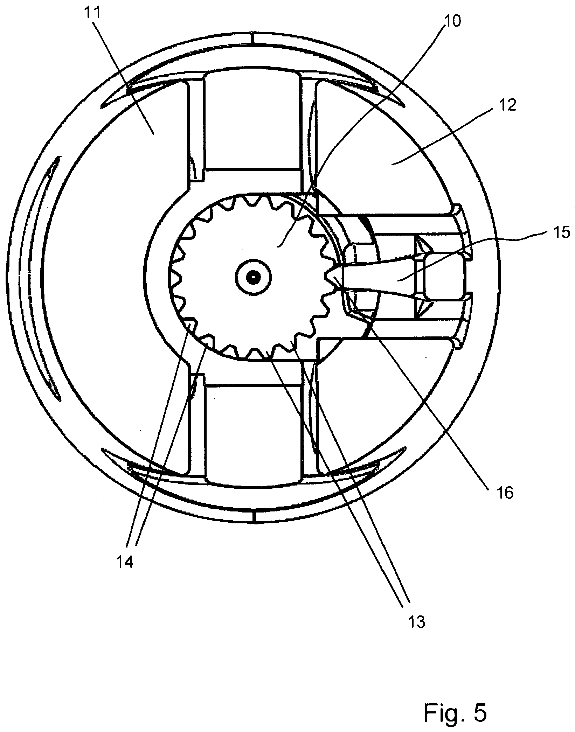

An den die Spiralnut 5 tragenden Grundkörper, zu dem die axiale Erstreckung d₁ definiert ist, schließt sich ein vom Grundkörper verschiedener zylindrischer Abschnitt 10 an, der einen Zahnkranz mit Zähnen 13 trägt, zwischen denen sich Zwischenräume in Form axialer Nuten 14 befinden, s. Fig. 3 bis 5. Am distalen Teil 4 ist eine Lamelle 15 angeordnet, deren Spitze 16 in die Zwischenräume 14 zwischen den Zähnen 13 eingreift. Wird das distale Teil 4 gegenüber dem proximalen Teil 2 gedreht, dann wird die Metalllamelle 15 elastisch gebogen, bis ihre Spitze 16 einen Zwischenraum 14 verlässt und in den nächsten, benachbarten Zwischenraum 14 einrastet. Auf diese Weise kann das distale Teil 4 stabil gegenüber dem proximalen Teil 2 gehalten werden und dennoch eine Einstichtiefe der Kanüle 3, also das Ausmaß d₃ ihres Herausstehens, sukzessive vergrößert oder verkleinert werden. Insbesondere kann die Situation an den zu spritzenden Patienten angepasst werden.

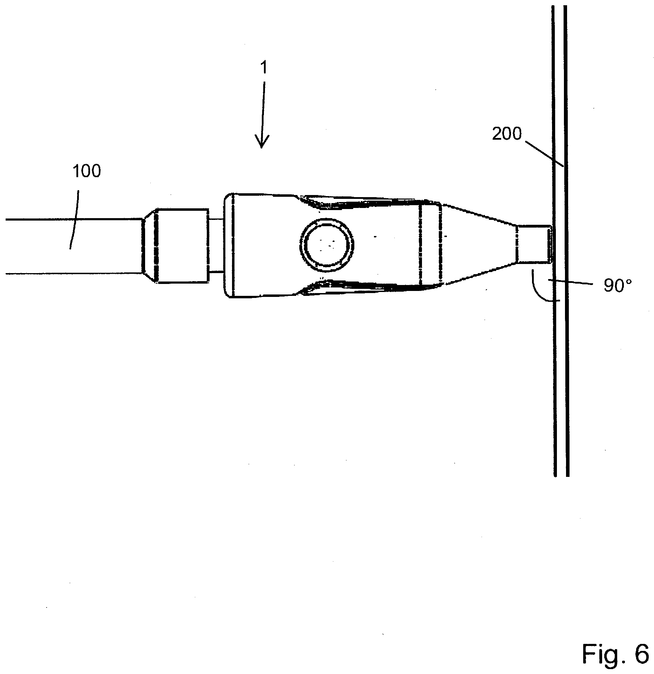

Die Fig. 6 zeigt den Aufsatz 10 mit daran angekoppeltem Spritzenkörper 100 als einer Vorrichtung zum Injizieren von Flüssigkeit in oder unter die Haut, insbesondere von Botulinumtoxin in Gesichtsmuskeln eines menschlichen Patienten. Die Haut ist in Fig. 6 schematisch durch eine Ebene 200 angedeutet. Die Spritze wird mit Hilfe eines an dem proximalen Teil 2 vorgesehenen Luer Lock ® 33 (siehe Fig. 1, Fig. 2) an dem Aufsatz 1 befestigt.

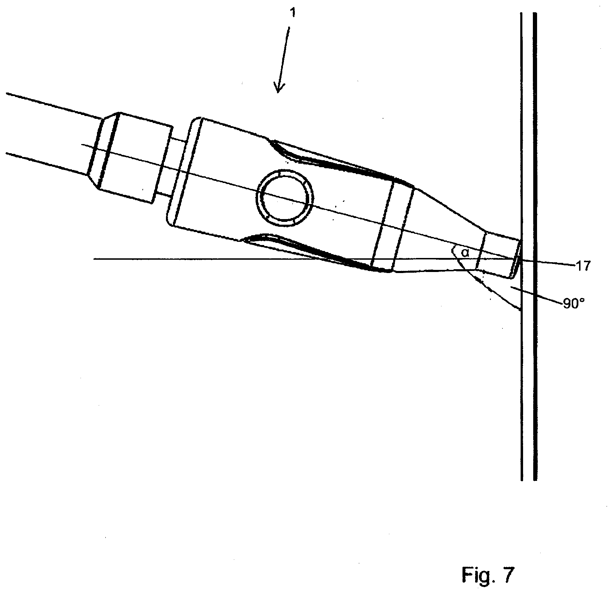

Die in den Fig. 1 und 2 teilweise sichtbare Stirnfläche 17 kann flach oder konkav oder konvex gewölbt sein, wobei auch bei einer Wölbung ein relativ kleiner Winkel von unter 15° am Rand gegenüber den Bereich um den Austrittspunkt der Kanüle 3 herum vorgesehen ist. Wie aus den Fig. 6 und 7 zu sehen, kann dadurch die Spritze 100 mit dem Aufsatz 1 stabil in einem Winkel von 90° aufgesetzt werden oder geringfügig um einen Winkel a, der höchstens 45° beträgt, von 90° abweichen.

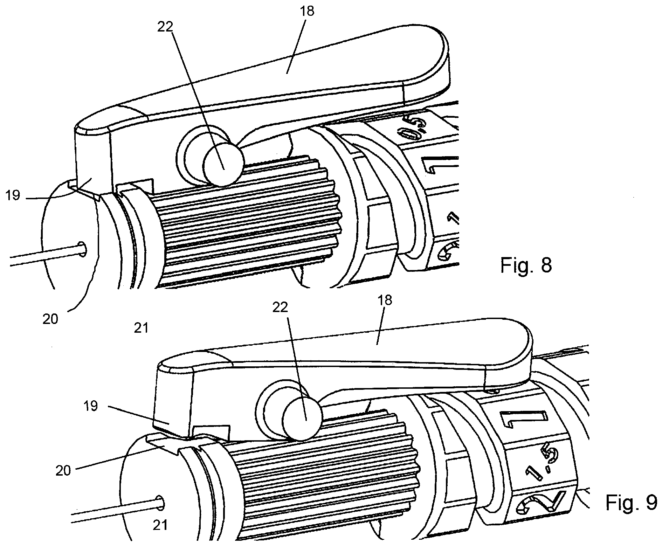

Insbesondere dann, wenn die Situation gemäß Fig. 1 gegeben ist, also die Kanüle 3 nicht aus dem distalen Teil 4 herausragt, ist es erwünscht, dass das distale Teil 4 stabil gegenüber dem proximalen Teil 2 verrastet, was einen Nadelstichschutz während des Aufsteckens der Spritze sowie nach dem Zurückfahren der Kanüle bietet. Die Fig. 8 zeigt hierzu einen Ausschnitt des proximalen Teils mit einer Arretierungstaste 18, welche zum distalen Teil gehört, das im Übrigen in Fig. 8 nicht gezeigt ist. Durch Betätigen der Arretierungstaste 18 kann ein Rastvorsprung 19 freigegeben werden, der in einer Aussparung 20 eines Tellers 21 eingreifen kann. Durch Betätigen der Arretierungstaste 18, bei der sich selbige um eine Welle 22 dreht, kann die in Fig. 9 gezeigte Freigabestellung erzielt werden und das distale Teil 4 gegenüber dem proximalen Teil 2 gedreht werden, um die Kanüle herauszufahren.

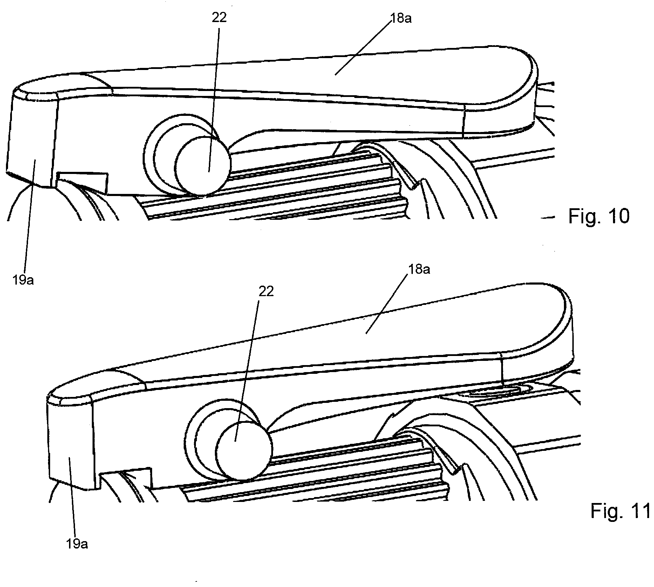

Während im Falle der Fig. 8 und 9 durch den Rastvorsprung 19 eine Drehbewegung des distalen Teils gegenüber dem proximalen Teil unterbunden ist, ist im Falle der Ausführungsformen gemäß Fig. 10 und 11 eine axiale Bewegung des distalen Teils gegenüber dem proximalen Teil unterbunden, was einen Nadelstichschutz während des Aufsteckens der Spritze sowie nach dem Zurückfahren der Kanüle bietet.

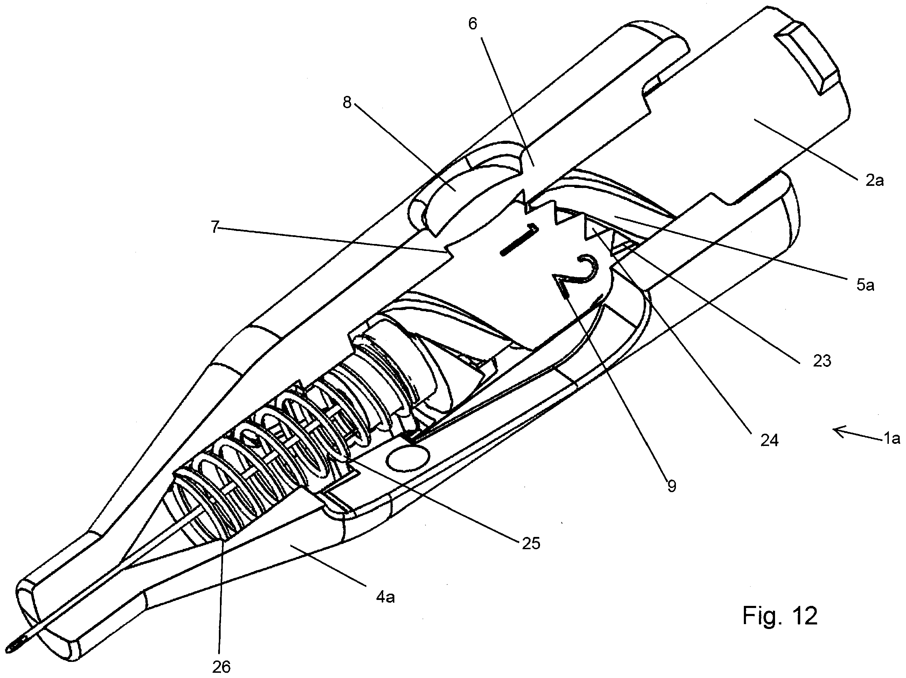

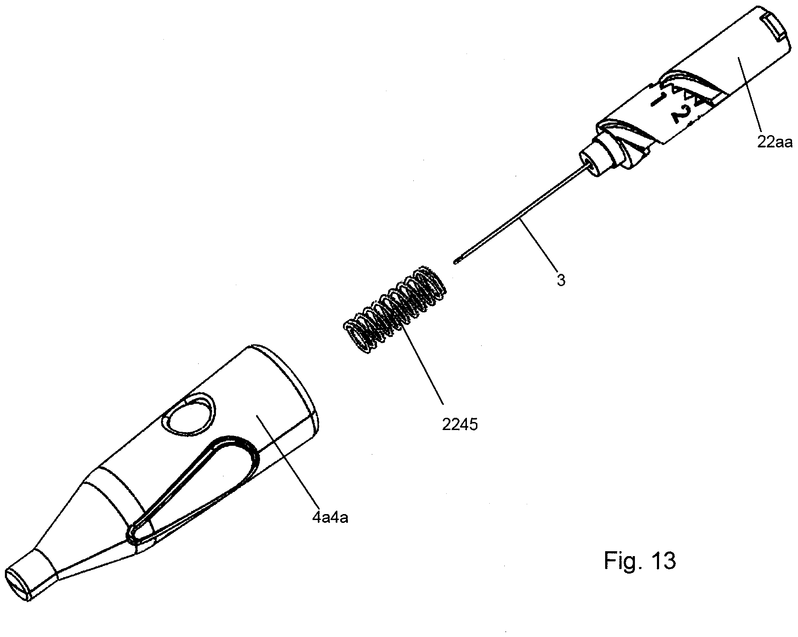

Fig. 12 zeigt ein zweites, nicht beanspruchtes Ausführungsbeispiel eines Aufsatzes 1a, bei dem das proximale Teil 2a im Bereich seiner Nut 5a Aussparungen 24 aufweist, die dreieckig sind, so dass sich eine Folge von Zähnen 23 ergibt, in die die Führungsspitze 6 einrasten kann. Hier ist somit durch die Nut 5a selbst die Rastmöglichkeit vorgegeben. Der zylindrische Körper 10 mit dem Zahnkranz ist nicht mehr erforderlich, und stattdessen ist ein Federelement 25 vorgesehen, welches sich an einem ringförmigen Sitz 26 des distalen Teils 4a abstützt. Fig. 13 zeigt diese Ausführungsform in Explosionsansicht.

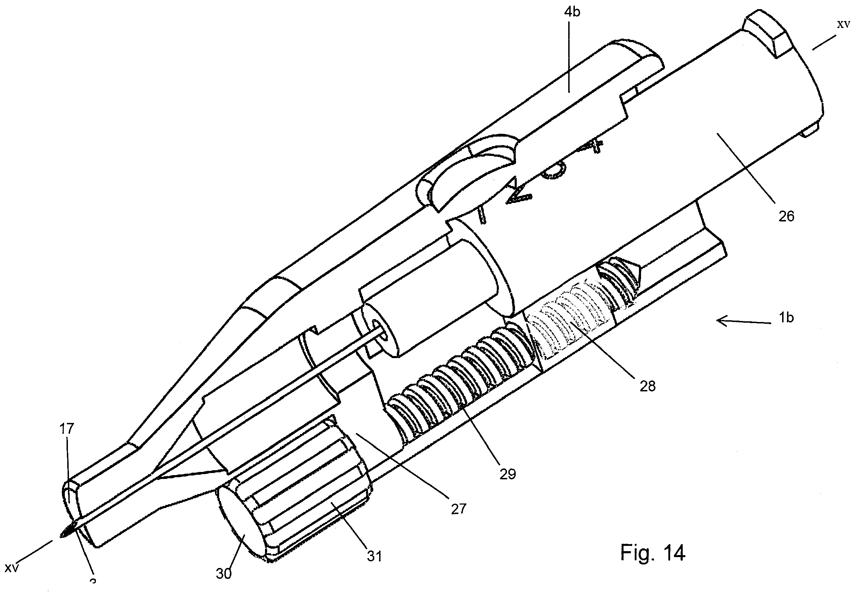

Fig. 14 zeigt ein drittes nicht beanspruchtes Ausführungsbeispiel eines Aufsatzes 1b, bei dem an das proximale Teil 2b ein Ansatz 28 angeformt ist und an das distale Teil 4b ein Ansatz 27 angeformt ist. Eine Stellschraube 29 ist durch beide Ansätze 27 und 28 geführt und greift insbesondere in ein in Fig. 14 durch Strichlierung angedeutetes Innengewinde des Ansatzes 28 ein. Gegebenenfalls kann auch im Ansatz 27 ein Innengewinde vorgesehen sein. Ein Kopf 30 der Stellschraube 29 ist mit einer Riffelung 31 zur leichteren Handhabung versehen: Eine Bedienperson kann an dem Kopf 30 drehen und dadurch bewirken, dass die Stellschraube das proximale Teil 2b axial gegenüber dem distalen Teil 4b verschiebt. Diese translatorische Bewegung hat etwa zur Folge, dass die Kanüle 3 aus der Stirnfläche 17 des distalen Teils 4b austritt.

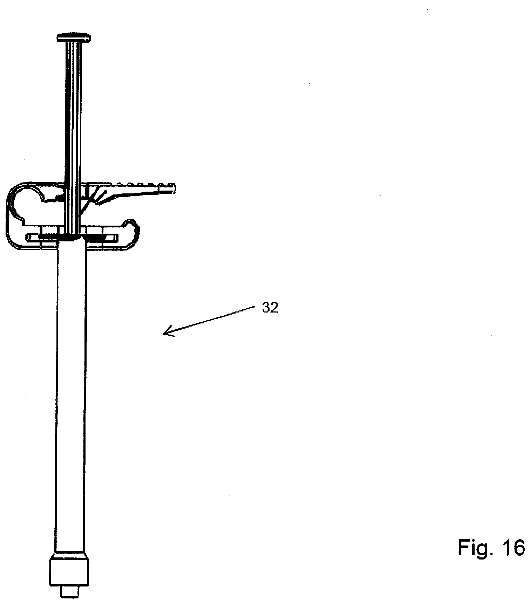

Der Aufsatz 1 kann außer über dem Luer Lock ® 33 mit einer Spritze verbunden sein oder auch stattdessen in einen Ultraschallkopf zur Beobachtung der Stelle, wo die Spritze eingebracht werden soll, integriert sein. Im Falle der Fig. 16 ist ein Dosier-Regler-Aufsatz für eine Spritze mit dem Aufsatz 1 oder 1b gemäß einer der Ausführungsformen der Erfindungen kombiniert.

Bei allen Ausführungsformen kann das distale Teil 4 oder 4a, 4b aus sehr weichem Kunststoff bestehen, wodurch ein Zusammendrücken des distalen Teils und ein unmittelbarer Halt des proximalen Teils 2, 2a, 2b ermöglicht ist. Vorzugsweise ist im Bereich der Stirnfläche 17 der zylindrische Mantel 34 transparent, so dass man das Ende der Kanüle 3 auch beim Vorgang des Spritzens erkennen kann. Die Kanüle 3 ist geeignet abgeschrägt, vergleiche zum Beispiel wie in Fig. 14 zu sehen, um ein präzises Setzen der Kanüle in oder unter die Haut oder Gesichtshaut eines menschlichen Patienten und weiter dann in einen Gesichtsmuskel, in die Subkutis oder ein anderes Organ oder Gewebe zu erleichtern.

Die in der vorstehenden Beschreibung, den Ansprüchen und den Zeichnungen offenbarten Merkmale können sowohl einzeln als auch in beliebiger Kombination für die Verwirklichung der Erfindung in ihren verschiedenen Ausgestaltungen von Bedeutung sein.

## Patentansprüche

1. Aufsatz (1, 1a) für eine oder an einer Vorrichtung (100) zum Injizieren einer Flüssigkeit in oder unter die Haut (200), wobei der Aufsatz (1, 1a) aufweist: ein proximales Teil (2, 2a), das an die Vorrichtung (100) ankoppelbar oder mit dieser verbunden ist, und das eine Kanüle (3) trägt, ein distales Teil (4, 4a) das die Kanüle (3) zumindest abschnittsweise umgibt und wobei das proximale Teil (2, 2a) und das distale Teil (4, 4a) derart zueinander drehbeweglich sind, dass ein Ausmaß (d3) des Herausragens der Kanüle (3) aus dem distalen Teil (4, 4a) veränderbar ist, wobei eine diskrete Anzahl an Haltestellungen bezüglich der Drehbewegung bereitgestellt ist, denen ein unterschiedliches Ausmaß des Herausragens der Kanüle (3) aus dem distalen Teil (4, 4a) entspricht, **dadurch gekennzeichnet, dass** der Aufsatz einen Rastmechanismus (10) mit einem Zahnkranz aus axial verlaufenden Zähnen (13) mit dazwischen axial verlaufenden Nuten (14) und mit einem elastischen Rastglied (15), das in einer Raststellung in einer der Nuten (14) eingreift, aufweist, die diskrete Anzahl an Haltestellungen durch eine Mehrzahl von Raststellungen bereitzustellen.

2. Aufsatz (1) nach Anspruch 1, **dadurch gekennzeichnet, dass** die Raststellungen beim Drehen des distalen Teils (4, 4a) in dieselbe Drehrichtung sukzessive durchlaufen werden und sich hierbei das Ausmaß (d3) des Herausragens der Kanüle (3) entweder ständig weiter vergrößert oder ständig weiter verkleinert.

3. Aufsatz (1) nach Anspruch 2, **dadurch gekennzeichnet, dass** das elastische Rastglied (15) als Lamelle ausgebildet ist.

4. Aufsatz (1) nach Anspruch 3, **dadurch gekennzeichnet, dass** der Zahnkranz (13) an dem proximalen Teil (2) ausgebildet ist und näher an der Kanüle (3) angeordnet ist als ein Gewinde (5), das die Drehbewegung des distalen Teils (4) gegenüber dem proximalen Teil (2) ermöglicht.

5. Aufsatz (1) nach Anspruch 3, **dadurch gekennzeichnet, dass** der Zahnkranz an einer Innenseite des distalen Teils ausgebildet ist.

6. Aufsatz (1, 1a) nach einem der Ansprüche 1 bis 5, **dadurch gekennzeichnet, dass** außen an dem proximalen Teil (2, 2a) eine Spiralnut (5, 5a) vorgesehen ist, in die das distale Teil (4, 4a) eingreift.

7. Aufsatz (1) nach Anspruch 6 in dessen Rückbezug auf Anspruch 5, **dadurch gekennzeichnet, dass** das distale Teil (4) und das proximale Teil (2) jeweils einen kreisrunden Querschnitt aufweisen, dass der Zahnkranz (13) eine maximale radiale Erstreckung hat, welcher kleiner ist als das radiale Abstand eines Grundes der Spiralnut (5) vom Mittelpunkt des kreisrunden Querschnitts.

8. Aufsatz (1, 1a) nach Anspruch 6 oder Anspruch 7, **dadurch gekennzeichnet, dass** zwischen verschiedenen Abschnitten der Spiralnut (5, 5a) ein eine Information tragender Abschnitt (9) angeordnet ist, mit zumindest einem Element aus einer Zahl, einem Buchstaben, einem Symbol, einem Skalenstrich, einem Grauwert auf einer Grauskala oder einer Farbe auf einer Farbskala, oder mit einer Kombination aus einem oder mehreren dieser Elemente.

9. Aufsatz nach einem der Ansprüche 1 bis 5, **dadurch gekennzeichnet, dass** in einem axial zur distalen Seite hin offenen Hohlraum des proximalen Teils ein Innengewinde ausgebildet ist, in das ein Schaft des distalen Teils eingreift.

10. Aufsatz nach Anspruch 1, **dadurch gekennzeichnet, dass** eine Mehrzahl von Zähnen mit Zwischenräumen bereitgestellt ist, in die ein elastisches Rastglied eingreift, wobei das Rastglied, wenn es eine Raststellung einnimmt, in axialer Richtung elastisch bewegbar ist.

11. Aufsatz (1a) nach Anspruch 1, **dadurch gekennzeichnet, dass** außen an dem proximalen Teil (2a) eine Spiralnut (5a) vorgesehen ist, in die das distale Teil (4a) eingreift, wobei an oder in der Spiralnut (5a) Rastvertiefungen (24) ausgebildet sind.

12. Aufsatz (1) nach einem der vorhergehenden Ansprüche, **gekennzeichnet durch** eine Einrichtung (18, 18a) an dem distalen Teil (4), mit Hilfe der es an dem proximalen Teil (2) in einer vorbestimmten Stellung festhaltbar ist, in der die Kanüle (3) gar nicht aus dem distalen Teil (4) herausragt, um für einen Nadelstichschutz vor der Injektion beim Aufsetzen der Vorrichtung (100) zum Injizieren oder nach einem Einfahren der Kanüle oder bei einem Wechsel der Vorrichtung (100) zum Injizieren zu sorgen.

13. Aufsatz (1, 1a, 1b) nach einem der vorhergehenden Ansprüche, **dadurch gekennzeichnet, dass** das distale Teil (4, 4a, 4b) zumindest einen Abschnitt aus einem elastisch nachgebenden Material umfasst, so dass durch einen Benutzer das distale Teil (4, 4a, 4b) soweit zusammendrückbar ist, dass das proximale Teil fassbar ist.

14. Aufsatz (1, 1a, 1b) nach einem der vorhergehenden Ansprüche, **dadurch gekennzeichnet, dass** das distale Teil (4, 4a, 4b) eine Stirnfläche (17) aufweist, welche flach oder gewölbt ist, vorzugsweise im Ganzen konvex oder im Ganzen konkav ist.

15. Aufsatz (1, 1a, 1b) nach einem der Ansprüche 1 bis 13, **dadurch gekennzeichnet, dass** das distale Teil (4, 4a, 4b) an seinem distalen Ende konisch zuläuft.

16. Aufsatz (1, 1a, 1b) nach einem der vorhergehenden Ansprüche, **dadurch gekennzeichnet, dass** das distale Teil an zumindest seinem distalen Ende (34) zumindest abschnittsweise transparent ist.

17. Aufsatz (1, 1a, 1b) nach einem der vorhergehenden Ansprüche, **dadurch gekennzeichnet, dass** ein transparentes Fenster, ein Vergrößerungsglas (7), eine Öffnung oder eine elektrisch betriebene Anzeige in bzw. an dem distalen Teil (4, 4a, 4b) bereitgestellt ist.

18. Aufsatz (1, 1a, 1b) nach einem der Ansprüche 1 bis 17, **dadurch gekennzeichnet, dass** der Aufsatz (1, 1a, 1b) an seinem proximalen Teil (2, 2a, 2b) eine Kupplung (33) zum Ankoppeln an die Vorrichtung (100) zum Injizieren einer Flüssigkeit aufweist, insbesondere in Form eines Konus zum Ankoppeln einer Spritze oder eines Schlauchanschlusses aufweist.

19. Aufsatz nach einem der Ansprüche 1 bis 17, **dadurch gekennzeichnet, dass** der Aufsatz mit der Vorrichtung zum Injizieren einer Flüssigkeit integral ist.

20. Aufsatz nach einem der Ansprüche 1 bis 17, **dadurch gekennzeichnet, dass** der Aufsatz in einen Ultraschallkopf für ein Ultraschallgerät oder eines Ultraschallgerätes integriert ist.

21. Aufsatz nach einem der Ansprüche 1 bis 17, **dadurch gekennzeichnet, dass** der Aufsatz in oder an einem Dosier-Regler-Aufsatz für eine Vorrichtung zum Injizieren an der Flüssigkeit ausgebildet ist.

## Claims

1. Attachment (1, 1a) for or on an apparatus (100) for injecting a liquid into or under the skin (200), wherein the attachment (1, 1a) comprises: a proximal part (2, 2a) which can be coupled to or connected to the apparatus (100) and which carries a cannula (3), a distal part (4, 4a) which surrounds the cannula (3) at least sectionwise, and wherein the proximal part (2, 2a) and the distal part (4, 4a) are rotationally movable relative to each other in such a way that an extent (d₃) of the protrusion of the cannula (3) from the distal part (4, 4a) is variable, wherein a discrete number of holding positions with respect to the rotational motion is provided, to which correspond a different extent of the protrusion of the cannula (3) from the distal part (4, 4a), **characterized in that** the attachment features a locking mechanism(10) with a gear rim of axially extending teeth (13) with axially extending grooves (14) therebetween and with an elastic locking member (15) engaging in a locking position in one of the grooves (14) to provide the discrete number of holding positions through a plurality of locking positions.

2. Attachment (1) according to claim 1, **characterized in that** the locking positions, when the distal part (4, 4a) rotates in the same direction of rotation, are successively passed through, and in this case the extent (d₃) of the protrusion of the cannula (3) either continuously increases further or continuously decreases further.

3. Attachment (1) according to claim 2, **characterized in that** the elastic locking member (15) is lamella-shaped.

4. Attachment (1) according to claim 3, **characterized in that** the gear rim (13) is formed on the proximal part (2) and disposed closer to the cannula (3) than a thread (5) which allows the rotational motion of the distal part (4) relative to the proximal part (2).

5. Attachment (1) according to claim 3, **characterized in that** the gear rim is formed on an interior side of the distal part.

6. Attachment (1, 1a) according to one of the claims 1 to 5, **characterized in that** a helical groove (5, 5a), into which the distal part (4, 4a) engages, is provided on the outside of the proximal part (2, 2a).

7. Attachment (1) according to claim 6, referring back to claim 5, **characterized in that** the distal part (4) and the proximal part (2) each feature a circular cross-section, **in that** the gear rim (13) has a maximum radial extension which is smaller than the radial distance of a base of the helical groove (5) from the center of the circular cross-section.

8. Attachment (1, 1a) according to claim 6 or claim 7, **characterized in that** between different portions of the helical groove (5, 5a) an information carrying portion (9) is disposed, with at least one element of a number, a letter, a symbol, a scale graduation mark, a gray value on a gray scale or a color on a color scale, or with a combination of one or more of these elements.

9. Attachment according to one of the claims 1 to 5, **characterized in that** an internal thread is formed in a cavity of the proximal part which is open axially towards the distal side and into which a shaft of the distal part engages.

10. Attachment according to claim 1, **characterized in that** a plurality of teeth with spaces are provided into which an elastic locking member engages, wherein the locking member is elastically movable in the axial direction when it assumes a locking position.

11. Attachment (1a) according to claim 1, **characterized in that** a helical groove (5a), in which the distal part (4a) engages, is provided on the outside of the proximal part (2a), wherein locking recesses (24) are formed on or in the helical groove (5a).

12. Attachment (1) according to one of the preceding claims, **characterized by** a means (18, 18a) on the distal part (4) for securing it to the proximal part (2) in a predetermined position in which the cannula (3) does not protrude from the distal part (4) at all to provide needle puncture protection prior to injection when the apparatus (100) for injecting is fitted or after retraction of the cannula or when the apparatus (100) for injecting is changed.

13. Attachment (1, 1a, 1b) according to one of the preceding claims, **characterized in that** the distal part (4, 4a, 4b) comprises at least one portion of an elastically yielding material, so that the distal part (4, 4a, 4b) can be compressed by a user to such an extent that the proximal part can be grasped.

14. Attachment (1, 1a, 1b) according to one of the preceding claims, **characterized in that** the distal part (4, 4a, 4b) features a front face (17) which is flat or curved, preferably convex as a whole or concave as a whole.

15. Attachment (1, 1a, 1b) according to one of the claims 1 to 13, **characterized in that** the distal part (4, 4a, 4b) tapers at its distal end.

16. Attachment (1, 1a, 1b) according to one of the preceding claims, **characterized in that** the distal part is at least partially transparent at at least its distal end (34).

17. Attachment (1, 1a, 1b) according to one of the preceding claims, **characterized in that** a transparent window, a magnifying glass (7), an opening or an electrically operated display is provided in or on the distal part (4, 4a, 4b).

18. Attachment (1, 1a, 1b) according to one of the claims 1 to 17, **characterized in that** the attachment (1, 1a, 1b) features on its proximal part (2, 2a, 2b) a coupling (33) for coupling to the apparatus (100) for injecting a liquid, in particular in the form of a cone for coupling a syringe or a hose connection.

19. Attachment according to one of the claims 1 to 17, **characterized in that** the attachment is integral with the apparatus for injecting a liquid.

20. Attachment according to one of the claims 1 to 17, **characterized in that** the attachment is integrated into an ultrasonic head for an ultrasonic apparatus or of an ultrasonic apparatus.

21. Attachment according to one of the claims 1 to 17, **characterized in that** the attachment is realized in or on a dosage controller attachment for an apparatus for injecting on the liquid.

## Revendications

1. Élément rapporté (1, 1a) pour ou sur un dispositif (100) pour injecter un liquide dans ou sous la peau (200), dans lequel l'élément rapporté (1, 1a) présente : une partie proximale (2, 2a) qui peut être couplée ou reliée au dispositif (100) et qui porte une canule (3), une partie distale (4, 4a) qui entoure la canule (3) au moins par sections, et dans lequel la partie proximale (2, 4a) et la partie distale (4, 4a) sont mobiles en rotation l'une par rapport à l'autre de telle sorte qu'une étendue (d₃) de la saillie de la canule (3) de la partie distale (4, 4a) est variable, dans lequel un nombre discret de positions d'arrêt par rapport au mouvement rotatif est prévu, auxquelles correspond une étendue différente de la saillie de la canule (3) de la partie distale (4, 4a), **caractérisé en ce que** l'élément rapporté présente un mécanisme de verrouillage (10) avec une couronne dentée formée de dents (13) s'étendant axialement avec des rainures (14) s'étendant axialement entre elles et avec un élément d'encliquetage élastique (15) qui s'engage dans une position encliquetée dans une des rainures (14) pour fournir le nombre distinct de positions d'arrêt par une pluralité de positions encliquetées.

2. Élément rapporté (1) selon la revendication 1, **caractérisé en ce que** les positions encliquetées, lorsque la partie distale (4, 4a) est tournée dans le même sens de rotation, sont traversées successivement, et dans ce cas, l'étendue (d₃) de la saillie de la canule (3) augmente ou diminue de manière continue.

3. Élément rapporté (1) selon la revendication 2, **caractérisé en ce que** l'élément d'encliquetage élastique (15) est réalisé sous forme d'une lamelle.

4. Élément rapporté (1) selon la revendication 3, **caractérisé en ce que** la couronne dentée (13) est formée sur la partie proximale (2) et est disposée plus près de la canule (3) qu'un filetage (5) qui permet le mouvement rotatif de la partie distale (4) par rapport à la partie proximale (2).

5. Élément rapporté (1) selon la revendication 3, **caractérisé en ce que** la couronne dentée est formée sur une face intérieure de la partie distale.

6. Élément rapporté (1, 1a) selon l'une des revendications 1 à 5, **caractérisé en ce qu'**une rainure hélicoïdale (5, 5a), dans laquelle s'engage la partie distale (4, 4a), est prévue à l'extérieur de la partie proximale (2, 2a).

7. Élément rapporté (1) selon la revendication 6, lorsqu'elle dépend de la revendication 5, **caractérisé en ce que** la partie distale (4) et la partie proximale (2) présente chacune une section transversale circulaire, **en ce que** la couronne dentée (13) a une extension radiale maximale qui est inférieure à la distance radiale d'une base de la rainure hélicoïdale (5) du centre de la section transversale circulaire.

8. Élément rapporté (1, 1a) selon la revendication 6 ou la revendication 7, **caractérisé en ce qu'**entre différentes sections de la rainure hélicoïdale (5, 5a) est disposée une section (9) portant des informations, avec au moins un élément d'un nombre, d'une lettre, d'un symbole, d'une ligne de graduation, d'une valeur de gris sur une échelle de gris ou d'une couleur sur une gamme de couleurs, ou avec une combinaison d'un ou plusieurs de ces éléments.

9. Élément rapporté selon l'une des revendications 1 à 5, **caractérisé en ce que**, dans une cavité de la partie proximale ouverte axialement vers le côté distal, un filet intérieur est formé dans lequel une tige de la partie distale s'engage.

10. Élément rapporté selon la revendication 1, **caractérisé en ce qu'**une pluralité de dents avec des espaces est prévue dans lesquels s'engage un élément d'encliquetage élastique, dans lequel l'élément d'encliquetage, lorsqu'il prend une position encliquetée, est mobile élastiquement dans la direction axiale.

11. Élément rapporté (1a) selon la revendication 1, **caractérisé en ce qu'**une rainure hélicoïdale (5a), dans laquelle s'engage la partie distale (4a), est prévue à l'extérieur de la partie proximale (2a), dans lequel des renfoncements d'encliquetage (24) sont formés sur ou dans la rainure hélicoïdale (5a).

12. Élément rapporté (1) selon l'une des revendications précédentes, **caractérisé par** un moyen (18, 18a) sur la partie distale (4) à l'aide duquel elle peut être fixée à la partie proximale (2) dans une position prédéterminée dans laquelle la canule (3) ne dépasse pas du tout de la partie distale (4) pour fournir une protection contre les piqûres d'aiguilles avant l'injection lorsque le dispositif (100) pour l'injection est monté ou après rétraction de la canule ou lorsque le dispositif (100) pour l'injection est changé.

13. Élément rapporté (1, 1a, 1b) selon l'une des revendications précédentes, **caractérisé en ce que** la partie distale (4, 4a, 4b) comprend au moins une partie d'un matériau élastiquement flexible, de sorte que la partie distale (4, 4a, 4b) peut être comprimée par un utilisateur dans une mesure telle que la partie proximale puisse être saisie.

14. Élément rapporté (1, 1a, 1b) selon l'une des revendications précédentes, **caractérisé en ce que** la partie distale (4, 4a, 4b) présente une face frontale (17) plate ou courbe, de préférence convexe dans son ensemble ou concave dans son ensemble.

15. Élément rapporté (1, 1a, 1b) selon l'une des revendications 1 à 13, **caractérisé en ce que** la partie distale (4, 4a, 4b) est conique à son extrémité distale.

16. Élément rapporté (1, 1a, 1b) selon l'une des revendications précédentes, **caractérisé en ce que** la partie distale est au moins partiellement transparente à au moins son extrémité distale (34).

17. Élément rapporté (1, 1a, 1b) selon l'une des revendications précédentes, **caractérisé en ce qu'**une fenêtre transparente, une loupe (7), une ouverture ou un affichage à commande électrique sont prévus dans ou sur la partie distale (4, 4a, 4b).

18. Élément rapporté (1, 1a, 1b) selon l'une des revendications 1 à 17, **caractérisé en ce que** l'élément rapporté (1, 1a, 1b) présente sur sa partie proximale (2, 2a, 2b) un embrayage (33) pour l'accouplement au dispositif (100) pour injecter un liquide, en particulier sous la forme d'un cône pour accoupler une seringue ou un raccord de tuyau.

19. Élément rapporté selon l'une des revendications 1 à 17, **caractérisé en ce que** l'élément rapporté est solidaire du dispositif d'injection d'un liquide.

20. Élément rapporté selon l'une des revendications 1 à 17, **caractérisé en ce que** l'élément rapporté est intégré dans une tête à ultrasons pour un appareil à ultrasons ou d'un appareil à ultrasons.

21. Élément rapporté selon l'une des revendications 1 à 17, **caractérisé en ce que** l'élément rapporté est formé dans ou sur un élément rapporté de contrôleur de dosage pour un dispositif d'injection sur le liquide.
